(19) Europäisches Patentamt
European Patent Office
Office européen des brevets

(11) Numéro de publication: **0 001 947**
**B1**

# (12) FASCICULE DE BREVET EUROPEEN

(45) Date de publication du fascicule de brevet: **14.10.81**

(21) Numéro de dépôt: **78400146.3**

(22) Date de dépôt: **24.10.78**

(51) Int. Cl.$^3$: **C 07 D 277/18, A 61 K 31/425**

## (54) Dérivés de l'amino-2 thiazoline, procédé de préparation et compositions pharmaceutiques les contenant.

(30) Priorité: **24.10.77 FR 7731933**

(43) Date de publication de la demande:
**16.05.79 Bulletin 79/10**

(45) Mention de la délivrance du brevet:
**14.10.81 Bulletin 81/41**

(84) Etats Contractants Désignés:
**BE CH DE GB LU NL SE**

(56) Documents cités:
**FR - A - 2 212 143**

(73) Titulaire: **INSTITUT MERIEUX Société anonyme dite:**
**17, rue Bourgelat**
**F-69002 LYON (FR)**

(72) Inventeur: **Boucherle André, Louis**
**116 Avenue de l'Eygala Corenc**
**F-38700 La Tronche (FR)**
Inventeur: **Viallet, Marie-Pierre, Danielle, née Guillaud**
**10, Place Jean-Moulin**
**F-38000 Grenoble (FR)**

(74) Mandataire: **Nony, Michel**
**Cabinet Nony 29, rue Cambacérès**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 001 947 B1

Dérivés de l'amino-2-thiazoline, procédé de préparation et compositions pharmaceutiques les contenant

La présente invention a pour objet les dérivés de l'amino-2 thiazoline, ainsi que les compositions pharmaceutiques les renfermant.

L'invention a plus précisément pour objet les dérivés de l'amino-2 thiazoline, caractérisés par le fait qu'ils répondent à la formule générale I:

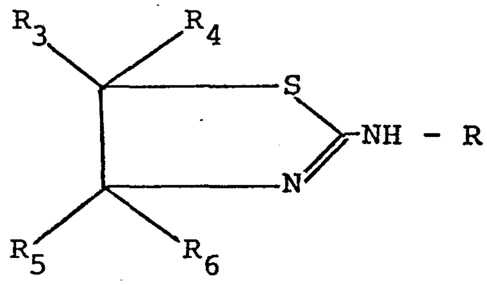

(I)

dans laquelle:

R représente un groupement —$R_1$ ou un groupement —CO—$R_2$, $R_1$ représente un groupement benzyle monosubstitué en ortho par un groupement alcoxy inférieur ou en ortho, méta ou para par un groupement alkyle inférieur, par un groupement trifluorométhyle ou par un atome d'halogène, ou $R_1$ représente un groupement $\alpha$-naphthylméthyle ou un groupement cycloalkylméthyle dont le groupement cycloalkyle à 5 ou 6 chaînons $R_2$ représente un groupement cycloalkyle, un groupement naphtyle, un groupement styryle ou un groupement phényle mono- ou disubstitue par un atome d'halogène, par un groupement alkyle ou alcoxy inférieur, par un groupement trifluorométhyle ou par un groupement nitro, et $R_3$, $R_4$, $R_5$ et $R_6$, égaux ou différents, représentent un atome d'hydrogène ou un groupement alkyle inférieur.

L'invention s'étend aux sels d'addition des amines de formule I avec les acides acceptables en pharmacie.

Dans la formule I, les substituants alkyle inférieur ou alcoxy inférieur ont de préférence 1 à 4 atomes de carbone; les substituants halogène sont le fluor, le chlore ou le brome; les substituants cycloalkyle ont de préférence 5 ou 6 chaînons; lorsque $R_2$ représente un radical phényle substitué, il s'agit de préférence d'un radical phényle mono- ou disubstitué par un atome d'halogène, un groupement alkyle, ou alcoxy inférieur, un groupement trifluorométhyle ou un groupement nitro; le ou les substituants pouvant occuper la position para, les positions méta ou de préférence les positions ortho.

L'invention concerne en particulier les dérivés de formule I dans lesquels $R_3 = R_4 = R_5 = R_6 =$ hydrogène ainsi que les dérivés de formule I dans lesquels l'un des couples ($R_3$, $R_4$) ou ($R_5$, $R_6$) représente un groupement

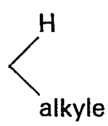

ou

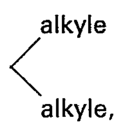

,

et l'autre représente deux atomes d'hydrogène.

Les composés de formule I, ainsi que, le cas échéant, leurs sels d'addition, possèdent des propriétés pharmacologiques intéressantes. Ils présentent notamment une activité anti-inflammatoire. Certains d'entre eux possèdent en outre une activité analgésique.

Ils sont utilisables par exemple dans le traitement des affections rhumatismales, des arthroses, des lumbagos, des algies musculaires, articulaires ou nerveuses, des douleurs dentaires, des zonas, des migraines, et aussi, à titre de traitements complémentaires, dans les états infectieux et fébriles.

La présente invention a donc pour objet, à titre de médicaments, les composés thérapeutiquement actifs de formule I, ainsi que leurs sels d'addition acceptables en pharmacie.

L'invention a en particulier pour objet, à titre de médicaments, les composés de formule I décrits ci-après dans les exemples 1 à 24.

Les sels d'addition pharmaceutiquement acceptables sont notamment ceux formés par les amines de formule I ($R = R_1$) avec les acides chlorhydrique, fumarique, maléique, oxalique, citrique ou succinique.

L'invention s'étend aux compositions pharmaceutiques renfermant comme principe actif au moins un des médicaments définis ci-dessus. La teneur en principe actif est généralement comprise entre 0,5 et 30% en poids.

Ces compositions peuvent être administrées, de préférence, par voie orale, rectale ou par voie locale en applications topiques sur la peau ou les muqueuses. A cet effet, elles peuvent être présentées sous forme de poudre, de granulés de tablettes, de comprimés, de pilules, de dragées, de gélules, de

solutions, suspensions ou émulsions buvables, de crèmes, de lotions, de pommades, ou de suppositoires.

Les compositions de l'invention contiennent, outre le principe actif, un excipient pharmaceutique permettant de présenter la composition sous une forme adaptée à l'administration par voie orale, rectale ou locale. L'excipient pharmaceutique comprend des agents diluants liquides (par exemple eau, alcool, huiles), des agents diluants solides pulvérulents, des agents liants, des agents lubrifiants, etc..., ou d'autres adjuvants communément utilisés, à condition qu'ils soient compatibles avec les dérivés de formule I. Parmi les agents diluants ou autres excipients communément utilisés, on citera par exemple la saccharose, le lactose, le glucose, l'amidon; parmi les agents liants on citera, par exemple la gélatine, la gomme arabique, la gomme adragante; parmi les agents lubrifiants on citera par exemple l'acide stéarique, le talc, le stéarate de magnésium, et analogues.

Ces formes pharmaceutiques sont préparées selon les méthodes usuelles.

La posologie varie notamment en fonction de la voie d'administration, du poids corporel et de l'effet thérapeutique recherché. Par exemple chez l'adulte elle peut varier entre 0,250 g et 3 g de principe actif par jour.

Dans un procédé de traitement contre la douleur et les maladies inflammatoires, on administre à un être humain ou à un animal, une quantité efficace d'au moins une amino-2 thiazoline de formule I telle que définie ci-dessus. On administre l'amino-2 thiazoline notamment sous la forme d'une composition telle que définie précédemment.

L'invention a également pour objet le procédé de préparation des composés de formule I.

Ce procédé est caractérisé par le fait que:

a) soit l'on fait agir sur une amino-2 thiazoline de formule II:

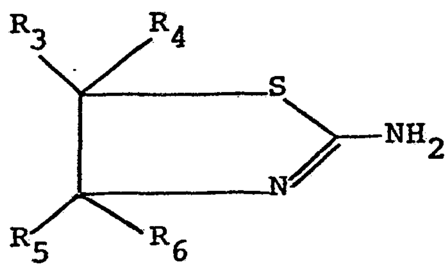

(II)

un chlorure d'acide de formule $R_2COCl$, à raison d'une mole de chlorure d'acide pour 2 moles d'amino-2 thiazoline, et isole l'amide obtenue de formule I (avec $R = —CO—R_2$).

b) soit l'on fait réagir l'amino-2 thiazoline de formule II avec un chlorure d'acide de formule $R'_1COCl$, $R'_1$ étant un groupement tel que le radical $—CH_2—R'_1$ est identique à $R_1$ tel que défini ci-dessus, à raison de 2 moles d'amino-2 thiazoline pour une mole de chlorure d'acide puis l'on soumet l'amide obtenue, de formule III suivante:

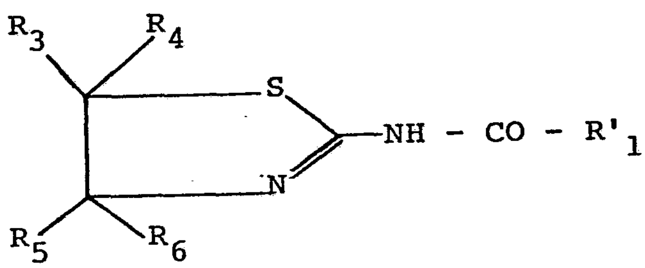

(III)

à l'action d'un agent réducteur capable de réduire le groupement carbonyle de l'amide en groupement méthylène, pour obtenir une amine de formule I (avec $R = R_1$),

c) soit l'on fait réagir un amino-alcool de formule IV

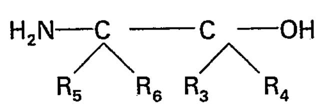

(IV)

dans laquelle $R_3$, $R_4$, $R_5$ et $R_6$ sont définis comme précédemment, avec un isothiocyanate de formule V

$$S=C=N—R_1 \qquad (V)$$

dans laquelle $R_1$ est défini comme précédemment pour obtenir une amine de formule I (avec $R = R_1$).

Les amino thiazolines de formule II peuvent être préparées notamment selon le procédé décrit dans le brevet des Etats-Unis n° 2.345.208.

L'action des chlorures d'acide sur l'amino-2 thiazoline est de préférence effectuée dans le benzène anhydre.

Pour réduire les amides III en l'amine correspondante on utilise de préférence comme agent réducteur l'hydrure de lithium et d'aluminium ($LiAlH_4$).

Les exemples suivants illustrent l'invention sans toutefois la limiter.

Les modes opératoires utilisés dans les exemples de préparation des produits de formule I sont les suivants:

a) pour préparer les amides de formule I on verse dans le récipient réactionnel 0,1 mole d'amino-2 thiazoline et 50 cm3 de benzène anhydre. On ajoute ensuite goutte à goutte, en agitant, 0,05 moles de chlorure d'acide dans 50 cm3 de benzène anhydre. Après addition complète, on maintient l'agitation pendant 4 heures à température ambiante. Dans certains cas, il est nécessaire de chauffer au reflux pendant quelques heures.

On sépare par filtration le précipité formé et le filtrat. On lave le précipité à l'eau distillée, le sèche et le purifie dans l'éthanol. On obtient l'amide correspondante.

On peut récupérer une partie de l'amide dans le filtrat en lavant celui-ci avec une solution de carbonate de sodium à 10% puis à l'eau, après quoi on sèche et évapore le solvant sous pression réduite. Le résidu purifié par recristallisation dans l'éthanol fournit un deuxième jet de l'amide désirée.

On a préparé de cette façon les amides suivantes:

TABLEAU I

| Exemples | Amide de formule I | PF en °C |
|---|---|---|
| 1 | (p-chlorobenzoyl) amino-2 thiazoline | 221 |
| 2 | (p-toluoyl) amino-2 thiazoline | 225 |
| 3 | (p-bromobenzoyl)amino-2 thiazoline | 221—222 |
| 4 | (m-toluoyl)amino-2 thiazoline | 160 |
| 5 | (dichloro-2′,6′ benzoyl)amino-2 thiazoline | 137 |
| 6 | (o-toluoyl)amino-2 thiazoline | 155—156 |
| 7 | (m-trifluorométhylbenzoyl)amino-2 thiazoline | 163—164 |
| 8 | (o-trifluorométhylbenzoyl)amino-2 thiazoline | 195 |
| 9 | (xyloyl-2′,6′)amino-2 thiazoline | 131—133 |
| 10 | (o-chlorobenzoyl) amino-2 thiazoline | 188,5—189 |
| 11 | (cyclohexylcarbonyl)amino-2 thiazoline | 172—173 |
| 12 | (naphtoyl-1)amino-2 thiazoline | 165—166 |
| 13 | (p-nitrobenzoyl)amino-2 thiazoline | 261—262 |
| 14 | (o-méthoxybenzoyl)amino-2 thiazoline | 154—154,5 |
| 15 | cinnamoyl amino-2 thiazoline | 168,5—169,5 |

Les amines de formule I décrites ci-après ont été préparées selon le mode opératoire suivant:

On mélange 0,025 mole d'aluminohydrure de lithium et 0,0125 mole d'amide dans 200 cm3 d'éther éthylique et on agite à la température du reflux pendant 2 heures. Après refroidissement on détruit l'excès d'hydrure par 30 cm3 d'eau, en refroidissant à 0°C.

On sépare le précipité par décantation, l'extrait à l'éther, réunit les phases éthérées, les sèche sur sulfate de sodium et les évapore sous pression réduite.

Le résidu est purifié par recristallisation dans l'hexane.

On a préparé de cette façon les amines suivantes:

TABLEAU II

| Exemples | Amine de formule I | PF en °C |
|---|---|---|
| 16 | (p-chlorobenzyl)amino-2 thiazoline | 108 |
| 17 | (p-méthylbenzyl)amino-2 thiazoline | 105 |
| 18 | (p-bromobenzyl)amino-2 thiazoline | 101 |
| 19 | (o-méthylbenzyl)amino-2 thiazoline | 123—123,5 |
| 20 | (m-trifluorométhylbenzyl)amino-2 thiazoline | 70—71 |
| 21 | (o-chlorobenzyl)amino-2 thiazoline | 104,5 |
| 22 | (cyclohexylméthyl)amino-2 thiazoline | 102—102,5 |
| 23 | ($\alpha$-naphtylméthyl)amino-2-thiazoline | 155,5—156,5 |
| 24 | (o-méthoxybenzyl)amino-2 thiazoline | 97—99 |

Pour préparer les sels d'addition de ces amines on opère selon les méthodes usuelles.

Comme indiqué ci-dessus les composés de formule I présentent des propriétés anti-inflammatoires et analgésiques qui ont été mises en évidence notamment sur les essais suivants:

— Test de l'abcès à la carrageenine: Benitz et Hall, Arch. Int. Pharmacod, 1963, *144*, 185.

— Test de l'oedème à la carrageenine: ce test est réalisé de la façon suivante.

— Le test de recherche de la protection vis-à-vis des crampes abdominales provoquées chez la souris pour l'injection intrapéritonéale de BPQ (phénylbenzoquinone). Ce test de l'activité analgésique est décrit par SIEGMUN) et coll., Proc. Soc. Exp. Biol. Med., 1957, *95*, 729—731.

Par injection de 0,05 ml d'une suspension aqueuse de carrageenine à 1% dans l'aponévrose plantaire de la patte postérieure gauche de rats mâles d'un poids moyen de 150 à 175 g, on provoque la formation d'un oedème. Le volume des pattes est mesuré à l'aide d'un pléthysmographe aux heures 0, 2, 4, 6 et 24, l'heure 0 étant l'heure de l'injection de carrageenine.

Les animaux traités reçoivent une heure avant l'injection de carrageenine (traitement préventif) ou deux heures après l'injection de carrageenine (traitement curatif) le produit étudié, par voie orale.

Les animaux témoins reçoivent uniquement le solvant dans les mêmes conditions.

On représente graphiquement l'évolution du volume des pattes en fonction du temps et on calcule pour chaque dose les pourcentages d'inhibition de l'oedème par rapport à l'oedème maximum observé chez les animaux témoins.

La recherche d'un éventuel effet ulcérogène a été effectuée selon le test décrit par P. Boissier et Coll., Thérapie, 1967, Tome 22, pages 157—168. Les produits de formule I ont un effet ulcérogène faible lorsqu'il est comparé par exemple avec celui de la phénylbutazone.

## EXEMPLES DE REALISATION DE COMPOSITIONS PHARMACEUTIQUES

### Exemple I

On a préparé des comprimés de 500 mg répondant à la formule suivante:

| | |
|---|---|
| — Produit de l'exemple 6 | 0,100—0,150 g |
| — Excipient (amidon, talc, stéarate de magnésium) qsp | 0,5 g |

### Exemple II

On a préparé une pommade pour application topique répondant à formule suivante:

| | |
|---|---|
| — Produit de l'exemple 18 | 1—1,5 g |
| — Excipient (lanoline et vaseline) qsp | 100 g |

**Revendications**

1. Nouveaux dérivés de l'amino-2 thiazoline, caractérisés par le fait qu'ils répondent à la formule générale I:

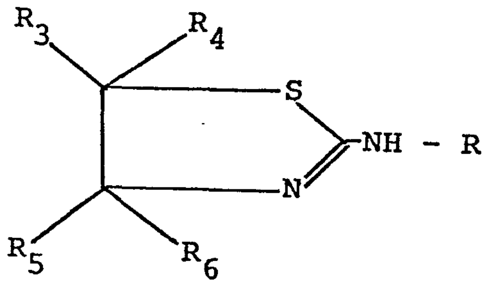

(I)

dans laquelle: R représente un groupement —$R_1$ ou un groupement —CO—$R_2$, $R_1$ représente un groupement benzyle monosubstitué en ortho par un groupement alcoxy inférieur ou en ortho, méta ou para par un groupement alkyle inférieur, par un groupement trifluorométhyle ou par un atome d'halogène, ou $R_1$ représente un groupement $\alpha$-naphtylméthyle ou un groupement cycloalkylméthyle dont le groupement cycloalkyle à 5 ou 6 chaînons, $R_2$ représente un groupement cycloalkyle, un groupement naphtyle, un groupement styryle, ou un groupement phényle mono- ou disubstitué par un atome d'halogène, par un groupement alkyle ou alcoxy inférieur, par un groupement trifluorométhyle ou par un groupement nitro, et $R_3$, $R_4$, $R_5$ et $R_6$, égaux ou différents, représentent un atome d'hydrogène ou un groupement alkyle inférieur.

2. Nouveaux dérivés selon la revendication 1, caractérisés par le fait que $R_3 = R_4 = R_5 = R_6 =$ hydrogène.

3. Nouveaux dérivés selon la revendication 1, caractérisés par le fait que l'un des couples ($R_3$, $R_4$) ou ($R_5$, $R_6$) représente un groupement

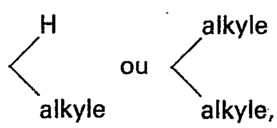

ou

et l'autre représente deux atomes d'hydrogène.

4. Nouveaux dérivés selon l'une quelconque des revendications 1 à 3, caractérisés par le fait que R représente $R_1$ tel que défini dans la revendication 1, ainsi que les sels d'addition de ses dérivés.

5. Nouveaux dérivés selon l'une quelconque des revendications 1 à 4, caractérisés par le fait que les substituants alkyle inférieur ou alcoxy inférieur ont de préférence 1 à 4 atomes de carbone, les substituants halogène sont le fluor, le chlore ou le brome, et les substituants cycloalkyle ont 5 ou 6 chaînons.

6. Nouveaux dérivés selon l'une quelconque des revendications 1 à 4, caractérisés par le fait que $R_2$ représente un radical phényle mono- ou disubstitué par un atome d'halogène, par un groupement alkyle ou alcoxy inférieur, par un groupement trifluorométhyle ou par un groupement nitro.

7. Nouveaux dérivés selon la revendication 6, caractérisés par le fait que le ou les substituants occupent la ou les positions ortho.

8. Nouveaux dérivés selon la revendication 1, caractérisés par le fait qu'ils sont choisis dans le groupe constitué par les composés suivants: (p-chlorobenzoyl)amino-2 thiazoline; (p-toluoyl)amino-2 thiazoline; (p-bromobenzoyl)amino-2 thiazoline; (m-toluoyl)amino-2 thiazoline; (dichloro-2',6'-benzoyl) amino-2 thiazoline; (o-toluoyl) amino-2 thiazoline; (m-trifluorométhylbenzoyl)amino-2 thiazoline; (o-trifluorométhylbenzoyl) amino-2 thiazoline; (xyloyl-2',6')amino-2 thiazoline; (o-chlorobenzoyl)amino-2 thiazoline; (cyclohexylcarbonyl)amino-2 thiazoline; (naphtoyl)amino-2 thiazoline; (p-nitrobenzoyl) amino-2 thiazoline; (o-méthoxybenzoyl)amino-2 thiazoline; cinnamoylamino-2 thiazoline.

9. Nouveaux dérivés selon la revendication 1, caractérisés par le fait qu'ils sont choisis parmi les composés suivants: (p-chlorobenzyl)amino-2 thiazoline; (p-méthylbenzyl)amino-2 thiazoline; (p-bromobenzyl)amino-2 thiazoline; (o-méthylbenzyl) amino-2 thiazoline; (m-trifluorométhylbenzyl) amino-2 thiazoline; (o-chlorobenzyl)amino-2 thiazoline; (cyclohexylméthyl) amino-2 thiazoline; ($\alpha$-naphtylméthyl) amino-2 thiazoline; (o-méthoxybenzyl)amino-2 thiazoline, ainsi que les sels d'addition de ces composés.

10. Procédé de préparation des dérivés de formule I tels que définis dans l'une quelconque des revendications précédentes, caractérisé par le fait que:
a) soit l'on fait agir sur une amino-2 thiazoline de formule II:

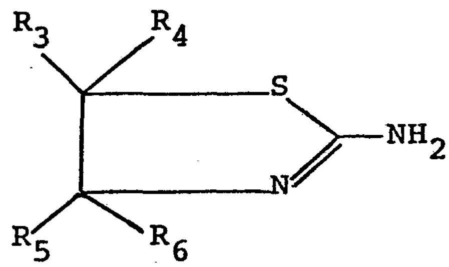

(II)

dans laquelle $R_3$, $R_4$, $R_5$ et $R_6$ sont définis comme précédemment, un chlorure d'acide de formule $R_2COCl$, à raison d'une mole de chlorure d'acide pour 2 moles d'amino thiazoline, et isole l'amide obtenue de formule I (avec $R = —CO—R_2$).

b) soit l'on fait réagir l'amino-2 thiazoline de formule II avec un chlorure d'acide de formule $R'_1COCl$, $R'_1$ étant un groupement tel que le radical $—CH_2—R'_1$ est identique à $R_1$ tel que défini ci-dessus, à raison de 2 moles d'amino-2 thiazoline pour une mole de chlorure d'acide, puis l'on soumet l'amide obtenue, de formule III suivante:

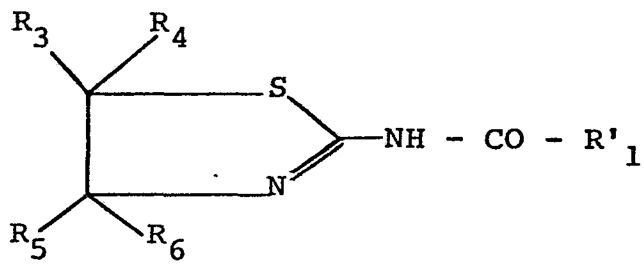

(III)

à l'action d'un agent réducteur capable de réduire le groupement carbonyle de l'amide en groupement méthylène, pour obtenir une amine de formule I (avec $R = R_1$),

c) soit l'on fait réagir un amino-alcool de formule IV

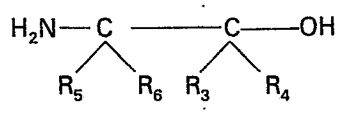

(IV)

dans laquelle $R_3$, $R_4$, $R_5$ et $R_6$ sont définis comme précédemment, avec un isothiocyanate de formule V

$$S{=}C{=}N{-}R_1 \qquad (V)$$

dans laquelle $R_1$ est défini comme précédemment pour obtenir une amine de formule I (avec $R = R_1$).

11. A titre de médicaments, les composés thérapeutiquement actifs selon l'une quelconque des revendications 1 à 9.

12. Compositions pharmaceutiques, caractérisées par le fait qu'elles renferment, à titre de principe actif au moins un médicament tel que défini dans la revendication 11.

## Patentansprüche

1. Neue Derivate von 1-Amino-2-thiazolin, dadurch gekennzeichnet, daß sie der allgemeinen Formel I entsprechen:

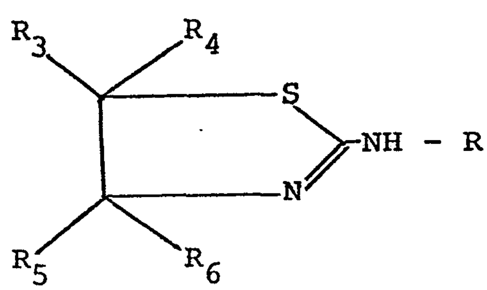

(I)

in der R eine Gruppe $—R_1$ oder eine Gruppe $—CO—R_2$ darstellt, wobei $R_1$ eine Benzylgruppe darstellt, die in ortho-Stellung durch eine niedere Alkoxygruppe oder in ortho-, meta- oder para-Stellung durch eine niedere Alkylgruppe, durch eine Trifluormethylgruppe oder durch ein Halogenatom substituiert ist oder $R_1$ eine $\alpha$-Naphthylmethylgruppe oder eine Cycloalkylmethylgruppe darstellt, wobei die Cyclo alkylgruppe 5- oder 6-gliedrig ist, und wobei $R_2$ eine Cycloalkylgruppe, eine Naphthylgruppe, eine Styrylgruppe oder eine Phenylgruppe, die durch ein Halogenatom, eine niedere Alkyl- oder Alkoxygruppe, eine Trifluormethylgruppe oder eine Nitrogruppe mono- oder disubstituiert ist, darstellt und $R_3$,

$R_4$, $R_5$ und $R_6$ gleich oder verschieden sind und ein Wasserstoffatom oder eine niedere Alkylgruppe bedeuten.

2. Neue Derivate gemäß Anspruch 1, dadurch gekennzeichnet, daß $R_3 = R_4 = R_5 = R_6 =$ Wasserstoff ist.

3. Neue Derivate gemäß Anspruch 1, dadurch gekennzeichnet, daß eine der Paarungen ($R_3$, $R_4$) oder ($R_5$, $R_6$) eine Gruppe

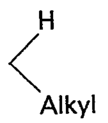 oder 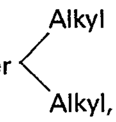

und die andere zwei Wasserstoffatome darstellt.

4. Neue Derivate nach einem der Ansprüche 1 bis 3, dadurch gekennzeichnet, daß R die Gruppe $R_1$ gemäß der Definition von Anspruch 1 darstellt, sowie die Additionssalze dieser Verbindungen.

5. Neue Derivate nach einem der Ansprüche 1 bis 4, dadurch gekennzeichnet, daß die niederen Alkylsubstituenten oder niederen Alkoxysubstituenten vorzugsweise 1 bis 4 Kohlenstoffatome aufweisen, daß die Halogensubstituenten Fluor, Chlor oder Brom sind und daß die Cycloalkylsubstituenten 5- oder 6-gliedrig sind.

6. Neue Derivate nach einem der Ansprüche 1 bis 4, dadurch gekennzeichnet, daß $R_2$ einen Phenylrest darstellt, der durch ein Halogenatom, eine niedere Alkyl- oder Alkoxygruppe, durch eine Trifluormethylgruppe oder durch eine Nitrogruppe mono- oder disubstituiert ist.

7. Neue Derivate gemäß Anspruch 6, dadurch gekennzeichnet, daß der oder die Substituenten die ortho-Stellung(en) besetzen.

8. Neue Derivate gemäß Anspruch 1, dadurch gekennzeichnet, daß sie ausgewählt sind aus der Gruppe bestehend aus folgenden Verbindungen: (p-Chlorbenzoyl)amino-2-thiazolin; (p-Toluoyl)-amino-2-thiazolin; (p-Brombenzoyl)amino-2-thiazolin; (m-Toluoyl)amino-2-thiazolin; (Dichlor-2′, 6′-benzoyl) amino-2-thiazolin; (o-Toluoyl) amino-2-thiazolin; (m-Trifluormethylbenzoyl)amino-2-thiazolin; (o-Trifluormethyl-benzoyl) amino-2-thiazolin; (Xyloyl-2′,6′) amino-2-thiazolin; (o-Chlorbenzoyl)amino-2-thiazolin; (Cyclohexylcarbonyl)amino-2-thiazolin; (Naphtoyl)amino-2-thiazolin; (p-Nitrobenzoyl) amino-2-thiazolin; (o-Methoxybenzoyl)amino-2-thiazolin; Cinnamoylamino-2-thiazolin.

9. Neue Derivate nach Anspruch 1, dadurch gekennzeichnet, daß sie ausgewählt sind aus der Gruppe bestehend aus folgenden Verbindungen: (p-Chlorbenzyl)amino-2-thiazolin; (p-Methylbenzyl)-amino-2-thiazolin; (p-Brombenzyl)amino-2-thiazolin; (o-Methylbenzyl)amino-2-thiazolin; (m-Trifluormethylbenzyl)amino-2-thiazolin; (o-Chlorbenzyl)amino-2-thiazolin; (Cyclohexylmethyl)amino-2-thiazolin; ($\alpha$-Naphtylmethyl)amino-2-thiazolin; (o-Methoxybenzyl)amino-2-thiazolin und den Additionssalzen dieser Verbindungen.

10. Verfahren zur Herstellung der Derivate der Formel I, wie sie in einem der vorstehenden Ansprüche definiert sind, dadurch gekennzeichnet, daß

a) man auf ein Amino-2-thiazolin der Formel II

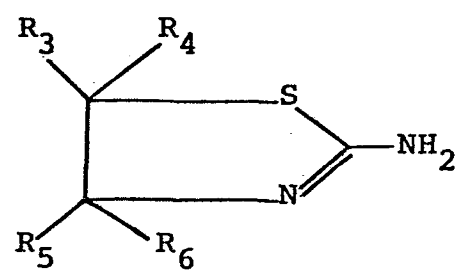

(II)

in der $R_3$, $R_4$, $R_5$ und $R_6$ wie vorher genannt definiert sind, auf ein Säurechlorid der Formel $R_2COCl$ einwirken läßt derart, daß ein Mol Säurechlorid mit 2 Molen Aminothiazolin reagieren, und das erhaltene Amid der Formel I (mit $R = -COR_2$) isoliert oder

b) daß man das Amino-2-thiazolin der Formel II mit einem Säurechlorid der Formel $R'_1COCl$, in der $R'_1$ eine Gruppe wie der Rest $-CH_2R'_1$ ist, der mit $R_1$ wie oben definiert identisch ist, umsetzt derart, daß 2 Mol Amino-2-thiazolin mit einem Mol Säurechlorid reagieren, und daß man das erhaltene Amid der folgenden Formel III

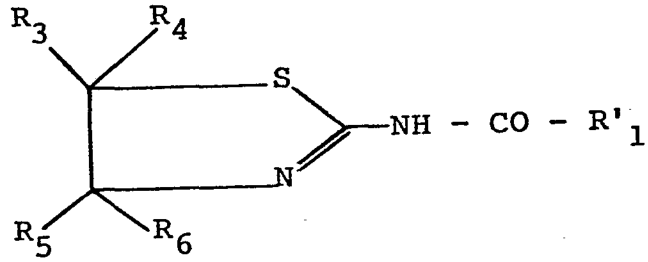

(III)

der Einwirkung eines Reduktionsmittels aussetzt, das in der Lage ist, die Carbonylgruppe des Amids zu einer Methylengruppe zu reduzieren, um ein Amin der Formel I (mit $R = R_1$) zu erhalten oder
c) daß man einen Aminoalkohol der Formel IV

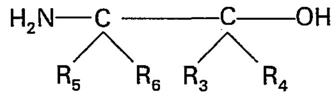

(IV)

in der $R_3$, $R_4$, $R_5$ und $R_6$ wie vorher definiert sind, mit einem Isothiocyanat der Formel V: $S = C = N—R_1$ in der $R_1$ wie oben definiert ist, umsetzt, um ein Amin der Formel I (mit $R = R_1$) zu erhalten.

11. Zur Verwendung als Arzneimittel die therapeutisch aktiven Verbindungen nach jedem der Ansprüche 1 bis 9.

12. Pharmazeutische Zubereitungen, dadurch gekennzeichnet, daß sie als Wirkstoff mindestens ein Arzneimittel enthalten, wie es in Anspruch 11 definiert ist.

## Claims

1. New 2-aminothiazoline derivatives, characterised in that they correspond to the general formula I:

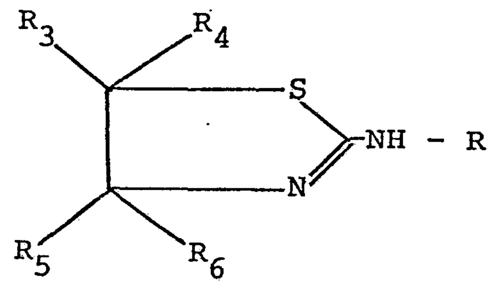

(I)

in which: R represents a group $—R_1$ or a group $—CO—R_2$, $R_1$ represents a benzyl group monosubstituted in the ortho-position by a lower alkoxy group or in the ortho-, meta- or para-position by a lower alkyl group, by a trifluoromethyl group or by a halogen atom, or $R_1$ represents an $\alpha$-naphthylmethyl group or a cycloalkylmethyl group in which the cycloalkyl group has 5 or 6 ring members, $R_2$ represents a cycloalkyl group, a naphthyl group; a styryl group or a phenyl group monosubstituted or disubstituted by a halogen atom, by a lower alkyl or alkoxy group, by a trifluoromethyl group or by a nitro group, and $R_3$, $R_4$, $R_5$ and $R_6$, which are identical or different, represent a hydrogen atom or a lower alkyl group.

2. New derivatives according to Claim 1, characterised in that $R_3 = R_4 = R_5 = R_6 =$ hydrogen.

3. New derivatives according to Claim 1, characterised in that one of the pairs ($R_3$, $R_4$) or ($R_5$, $R_6$) represents a group

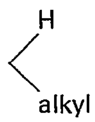 or 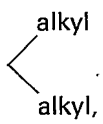

and the other represents two hydrogen atoms.

4. New derivatives according to any one of Claims 1 to 3, characterised in that R represents $R_1$ as defined in Claim 1, and also the addition salts of these derivatives.

5. New derivatives according to any one of Claims 1 to 4, characterised in that the lower alkyl or lower alkoxy substituents preferably have 1 to 4 carbon atoms, the halogen substituents are fluorine, chlorine or bromine and the cycloalkyl substituents have 5 or 6 ring members.

6. New derivatives according to any one of Claims 1 to 4, characterised in that $R_2$ represents a phenyl radical monosubstituted or disubstituted by a halogen atom, by a lower alkyl or alkoxy group, by a trifluoromethyl group or by a nitro group.

7. New derivatives according to Claim 6, characterised in that the substituent or substituents occupy the ortho-position or ortho-positions.

8. New derivatives according to Claim 1, characterised in that they are chosen from the group comprising the following compounds: 2-(p-chlorobenzoyl)-aminothiazoline, 2-(p-toluoyl)-aminothiazoline, 2-(p-bromobenzoyl)-aminothiazoline, 2-(m-toluoyl)-aminothiazoline, 2-(dichloro-2′,6′-benzoyl)-aminothiazoline, 2-(o-toluoyl)-aminothiazoline, 2-(m-trifluoromethylbenzoyl)-aminothiazoline, 2-(o-trifluoromethylbenzoyl)-aminothiazoline, 2-(2′,6′-xyloyl)-aminothiazoline, 2-(o-chlorobenzoyl)-aminothiazoline, 2-(cyclohexylcarbonyl)-aminothiazoline, 2-(naphthoyl)-aminothiazoline, 2-(p-nitrobenzoyl)-aminothiazoline, 2-(o-methoxybenzoyl)-aminothiazoline and 2-cinnamoyl-aminothiazoline.

9. New derivatives according to Claim 1, characterised in that they are chosen from amongst the following compounds: 2-(p-chlorobenzyl)-aminothiazoline, 2-(p-methylbenzyl)-aminothiazoline, 2-(p-bromobenzyl)-aminothiazoline, 2-(o-methylbenzyl)-aminothiazoline, 2-(m-trifluoromethylbenzyl)-aminothiazoline, 2-(o-chlorobenzyl)-aminothiazoline, 2-(cyclohexylmethyl)-aminothiazoline, 2-($\alpha$-naphthylmethyl)-aminothiazoline and 2-(o-methoxybenzyl)-aminothiazoline, and also the addition salts of these compounds.

10. Process for the preparation of the derivatives of the formula I as defined in any one of the preceding claims, characterised in that:

a) either an acid chloride of the formula $R_2COCl$ is reacted with a 2-aminothiazoline of the formula II

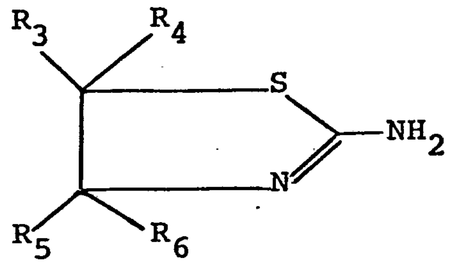

(II)

in which $R_3$, $R_4$, $R_5$ and $R_6$ are defined as above, at a rate of one mol of acid chloride per 2 mols of aminothiazoline, and the resulting amide of the formula (I) (in which $R = —CO—R_2$) is isolated,

b) or the 2-aminothiazoline of the formula II is reacted with an acid chloride of the formula $R'_1COCl$, $R'_1$ being a group such that the radical $—CH_2—R'_1$ is identical to $R_1$ as defined above, at a rate of 2 mols of 2-aminothiazoline per mol of acid chloride, and the resulting amide of the following formula III:

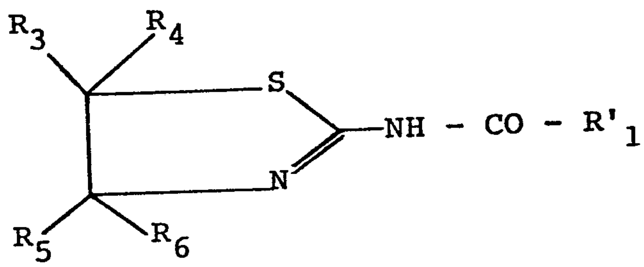

(III)

is subjected to the action of a reducing agent capable of reducing the carbonyl group of the amide to a methylene group, in order to obtain an amine of the formula I (in which $R = R_1$),

c) or an aminoalcohol of the formula IV

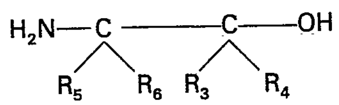

(IV)

in which $R_3$, $R_4$, $R_5$ and $R_6$ are defined as above, is reacted with an isothiocyanate of the formula V:

$$S = C = N—R_1$$

in which $R_1$ is defined as above, in order to obtain an amine of the formula I (in which $R = R_1$).

11. As medicaments, the therapeutically active compounds according to any one of Claims 1 to 9.

12. Pharmaceutical compositions, characterised in that they contain, as the active principle, at least one medicament as defined in Claim 11.